# EUROPÄISCHE PATENTSCHRIFT

(11) **EP 1 697 739 B1**
(45) Veröffentlichungstag und Bekanntmachung des Hinweises auf die Patenterteilung: **02.12.2009**
(21) Anmeldenummer: 04803562.0
(22) Anmeldetag: 06.12.2004
(51) Int. Cl.: G01N 33/18, G01N 33/00

(54) **VERFAHREN UND ANORDNUNG ZUR BESTIMMUNG VON WASSERINHALTSSTOFFEN**
METHOD AND ARRANGEMENT FOR DETERMINING CONSTITUENTS IN WATER
PROCEDE ET SYSTEME POUR DETERMINER DES SUBSTANCES CONTENUES DANS L'EAU

(30) Priorität: 22.12.2003 DE 10360445
(43) Veröffentlichungstag der Anmeldung: 06.09.2006
(73) Patentinhaber: LAR Process Analysers AG, 12057 Berlin (DE)
(72) Erfinder: ARTS, Werner, 10825 Berlin (DE); MARTENS, Berndt, 22941 Bargteheide (DE)
(74) Vertreter: Heinze, Ekkehard
(86) Internationale Anmeldenummer: PCT/EP2004/013865
(87) Internationale Veröffentlichungsnummer: WO 2005/064329

(56) Entgegenhaltungen:
- EP-A- 0 512 238
- EP-A- 0 730 153
- DE-A1- 2 362 773
- DE-A1- 4 309 646
- DE-C1- 10 012 730
- PATENT ABSTRACTS OF JAPAN Bd. 1997, Nr. 11, 28. November 1997 (1997-11-28) & JP 09 178723 A (SHIMADZU CORP), 11. Juli 1997 (1997-07-11)

## Beschreibung

Die Erfindung betrifft ein Verfahren zur Bestimmung von Wasserinhaltsstoffen gemäß dem Oberbegriff des Anspruchs 1 sowie eine Anordnung zur Durchführung des Verfahrens, gemäß dem Oberbegriff des Anspruchs 8.

Es ist bekannt, zur Bestimmung des Gehaltes an bestimmten Wasserinhaltsstoffen - und damit der Qualität von Wasser - eine Probe in einer Atmosphäre eines mit Sauerstoff angereicherten inerten Transportgases zu verdampfen und zu verbrennen und das hierbei erhaltene Verbrennungsgasgemisch einem zum Nachweis von Kohlendioxid, Stickoxiden etc. geeigneten Detektor zuzuführen. Als Detektoren haben sich (neben anderen) Infrarotdetektoren für den Kohlenstoffgehalt, spezielle Chemolumineszenzdetektoren für den Stickoxidgehalt und sogenannte coulometrische Detektoren für den Halogenidgehalt bewährt.

Große Verbreitung haben die auf der Verbrennung einer Wasserprobe beruhenden Nachweisverfahren für die Erfassung des Gehaltes an organischen Inhaltsstoffen - des sogenannten TOC (total organic carbon) - erlangt. Hierbei wird üblicherweise eine kleine Wassermenge mit dem Transportgas einem auf eine vorbestimmte Temperatur aufgeheizten Ofen zugeführt, wo sie nahezu schlagartig verdampft und verbrennt, und das Verbrennungsgas wird einem NDIR-CO₂-Detektor zugeführt. Dessen CO₂-Gehalts-Messergebnis stellt ein Maß für den C-Gehalt der Wasserprobe dar.

Eine Ausführung dieses Verfahrens und eine entsprechende Apparatur sind in DE 43 44 441 C2 beschrieben. Eine zur Messung sehr niedriger TOC-Werte - etwa in hochreinem Wasser bzw. hochreinen Lösungen für medizinische Anwendungen - modifizierte Anordnung ist in der EP 0 684 471 A2 beschrieben.

Mit diesen Verfahren wird nicht ohne weiteres der interessierende TOC, sondern grundsätzlich der Gesamt-Kohlenstoffgehalt des Wassers (TC = total carbon) bestimmt, der neben dem TOC den Anteil an anorganischen Kohlenstoffverbindungen (TIC = total inorganic carbon) umfasst. Diese werden daher zur Bestimmung des TOC in einem vorgeschalteten Abtrennschritt (sog. Strippen) abgetrennt; vgl. dazu etwa die DE 39 42 229 C2 (mit weiteren Literaturhinweisen).

Bei der Abtrennung des anorganischen Kohlenstoffs durch Austreiben tritt das weitere Problem auf, dass austreibbarer bzw. flüchtiger organischer Kohlenstoff (POC bzw. VOC = volatile organic carbon) ebenfalls aus der Probe entfernt wird. In DE 43 09 646 A1 werden daher ein Verfahren und eine Untersuchungsanordnung des oben skizzierten Typs vorgeschlagen, bei denen der POC-Gehalt getrennt gemessen und zur Gewinnung korrekter POC-Messwerte ungewollt mit ausgetriebenen Kohlenstoffverbindungen durch ein spezielles Adsorberagens abgefangen wird.

Bei Messverfahren und -vorrichtungen der genannten Art ist in bestimmten Abständen eine Kalibrierung mit Kalibrierproben erforderlich, die einen exakt bekannten Anteil des zu bestimmenden Inhaltsstoffes aufweisen, um beispielsweise Langzeitdrifts der Detektoren erfassen und - falls vorhanden - durch entsprechende Änderungen des Auswertungsalgorithmus kompensieren zu können. Es gibt industrielle Anwendungsfälle, in denen eine solche Kalibrierung in nicht zu langen Zeitabständen durchgeführt werden sollte, weil das in ihnen eingesetzte Wasser kompromisslos und zuverlässig höchsten Reinheitsanforderungen genügen muss. So ist beispielsweise in der pharmazeutischen Industrie eine mit Reinstwasser, dessen TOC-Gehalt einen bestimmten Grenzwert überschreitet, hergestellte Produktionscharge zu verwerfen. Bei Feststellung einer solchen Unregelmäßigkeit im Zuge einer Kalibrierung der Messapparatur muss dann die gesamte Produktionsmenge seit der letzten Kalibrierung verworfen werden, was zu sehr hohen finanziellen Einbußen beim Hersteller führen kann.

Zur Vermeidung zu starker stoßartiger Druckbelastungen der Analysenapparatur durch ein Verpuffen im heißen Ofen müssen die zugeführten Wasserprobenmengen sehr klein gemacht werden, was den Einsatz präzisester Dosiertechnik voraussetzt. Für die oben erwähnten Kalibrierungen mit Wasserproben, die einen sehr geringen und exakt definierten Anteil des zu erfassenden Wasserinhaltsstoffes enthalten (sogenannte "Nullwasserlösungen") wirft das zusätzliche Probleme auf: Jede kleinste Verunreinigung eines verwendeten Gefäßes, Dosiermittels etc. kann beim Kalibrierungsvorgang hochgradig verfälschend wirken. Dies verschärft die ohnehin problematische Situation, dass bei Nullwasser-Konzentrationen des relativen Inhaltsstoffes (z.B. Kohlenstoff) unterhalb von 1 mg/l ein exakter Ansatz der Kalibrierlösungen und speziell auch die exakt graduierte Einstellung verschiedener Inhaltsstoffe-Konzentrationen schon deshalb problematisch ist, weil das "Verdünnungswasser" ebenfalls nicht völlig rein ist.

Im übrigen sind die erwähnten Kalibrierungsprozeduren unter Einsatz von Kalibrier- bzw. Nullwasserlösungen auch arbeitsaufwendig und erfordern den Einsatz von qualifiziertem Fachpersonal, und hinzu kommt noch eine relativ große Störungsanfälligkeit aufgrund von Umgebungseinflüssen. Im Grunde ist im genannten Konzentrationsbereich die Gewährleistung von Reinstraumbedingungen erforderlich, einschließlich der entsprechenden Klimatechnik und Bekleidung des Personals.

Ein gattungsgemäßes Verfahren, bei dem der Detektor mithilfe einer Kalibriergasküvette kalibriert wird, ist aus der EP 0 512 238 bekannt.

Der Erfindung liegt deshalb die Aufgabe zug runde, ein kostengünstiges und für den Routinebetrieb geeignetes Verfahren mit hoher Messgenauigkeit zur spezifischen Bestimmung von Wasserinhaltsstoffen, speziell des TOC in hochreinem Wasser für pharmazeutische Prozesse, sowie eine Vorrichtung zur Durchführung des Verfahrens anzugeben.

Die Aufgabe wird hinsichtlich ihres Verfahrensaspekts durch ein Verfahren mit den Merkmalen des Anspruchs 1 und hinsichtlich des Vorrichtungsaspekts durch eine Vorrichtung mit den Merkmalen des Anspruchs 8 gelöst.

Die Erfindung schließt den wesentlichen Gedanken ein, eine Kalibrierung mit einem Kalibriergas auszuführen. Diese Idee fußt auf der Überlegung, dass auch ein Gas mit einer vorbestimmten Menge des zu erfassenden Inhaltsstoffes einer zu prüfenden Wasserprobe (etwa Kohlenstoff) dotiert werden kann. Weiterhin gehört hierzu die Überlegung, dass ein solches Kalibriergas mit präzise vorbestimmter Dotierung mit bekannten Technologien relativ leicht und kostengünstig herstellbar und im Laborbetrieb auf einfache Weise und hochgradig frei von Umwelteinflüssen handhabbar ist.

Mit der Erfindung werden insbesondere in industriellen Prozessen, in denen kontinuierlich Reinstwasser mit sehr geringer und strikt begrenzter Konzentration an bestimmten Inhaltsstoffen zur Verfügung stehen muss, erhebliche Vorteile erreicht: Die relativ leichte Handhabung ermöglicht in unaufwendiger Weise Kalibrierungen in kurzen Abständen, ohne dass hierzu aufwendige Maßnahmen zur Abschirmung von Umwelteinflüssen (Reinstraumbedingungen, spezielle Bekleidung und Verhaltensweisen des Personals und der Einsatz von qualifiziertem Analysepersonal) erforderlich wären. Zudem sind die gasförmigen Kalibrierproben - jedenfalls nach Etablierung des vorgeschlagenen Verfahrens - kostengünstig verfügbar.

In einer besonders bedeutsamen Ausführungsvariante des Verfahrens wird zur Bestimmung des Gehaltes von Messproben an organischem Kohlenstoff (TOC) ein Kalibriergas mit einem vorbestimmten CO₂-Gehalt eingesetzt. Diese Anwendung ist besonders wichtig in der pharmazeutischen Industrie, wo bei vielen Verfahren Reinstwasser mit einem streng limitierten TOC-Gehalt eingesetzt werden muss und Produkte, die unter Einsatz von Wasser mit erhöhtem TOC-Gehalt hergestellt wurden, zu verwerfen sind.

In einer bevorzugten Verfahrensführung wird eine vorbestimmte Menge des Kalibriergases durch Befüllung eines Reservoirs, insbesondere Schlauchabschnittes, mit bekanntem Volumen unter Atmosphärendruck oder mit Druckkompensation eingestellt, welches nach Befüllung mit dem Kalibriergas vom Transportgasstrom durchströmt. Hiermit werden an sich bekannte Loop-Injektionsverfahren und -anordnungen, bei denen auch die Messproben zunächst in einem entsprechenden Reservoir (Schlauchabschnitt) gesammelt und dann dem Verbrennungsofen zugeführt werden, erfindungsgemäß verbessert und die o.g. Vorteile erreicht.

In einer weiteren vorteilhaften Verfahrensführung ist vorgesehen, dass im Rahmen einer Kalibrierungsprozedur ein mehrmaliger Eintrag von Kalibriergas in das Verbrennungsgefäß, mit jeweiliger Erfassung des Wasserinhaltsstoffes im Detektor, erfolgt. Hiermit kann den üblichen Kalibrierungs-Vorschriften entsprochen werden, die eine statistische Auswertung über mehrere Messpunkte vorsehen. In einer Fortbildung dieser Ausführungsform zeichnet sich das Verfahren dadurch aus, dass eine mehrschrittige Kalibrierung mit einer Mehrzahl verschiedener Kalibriergase ausgeführt wird, welche unterschiedliche vorbestimmte Anteile des zu bestimmenden Elementes enthalten. Dies stellt eine besonders einfache, schnelle und wenig fehleranfällige Realisierung der (an sich bekannten und in DIN- bzw. EN-Vorschriften vorgeschriebene) Kalibrierung an mindestens fünf Konzentrations-Punkten auf einer linearen Geraden dar.

Was die Auswertung der erfindungsgemäßen Kalibrierungs-Messungen angeht, so wird in zweckmäßiger Weise die Fläche unter einen Messsignalpeak am Detektor integriert und auf den vorbestimmten Gehalt des Elementes im Kalibriergas normiert. Speziell erfolgt die Normierung unter Einsatz eines vorbestimmten Korrekturfaktors.

Die vorrichtungsseitigen Aspekte der Erfindung korrespondieren im wesentlichen zu den oben ausgeführten Verfah rensaspekten und werden insoweit hier nicht nochmals dargestellt. Die Bereitstellung der Kalibriergase erfolgt zweckmäßigerweise in industrieüblicher Verpackung, also in an sich bekannten Druckgasflaschen. Bei der oben erwähnten Loop-Variante ist mindestens eine solche Flasche an einen vorbestimmten Schlauchabschnitt der Apparatur an der Eingangsseite des Erhitzungsgefäßes (Verbrennungsofens) angeschlossen; zweckmäßigerweise sind zur Realisierung der ebenfalls oben erwähnten Mehrpunkt-Kalibrierung dort mehrere Gasflaschen angeschlossen, die den relevanten Inhaltsstoff in unterschiedlicher Konzentration (ppm, ppw) enthalten. Der Anschluss kann in einfacher Weise durch übliche Absperrventile bzw. die Absperr- und Reduzierventile der Gasflaschen, in Verbindung auch mit anordnungsseitigen Mehrwegeventilen, erfolgen.

Für den oben erwähnten wichtigen Einsatzfall der TOC-Bestimmung in Prozesswasser (Reinstwasser) ist die Anbindung von Kalibriergasflaschen an die Analysenanordnung vorgesehen, die CO₂ in vorbestimmter Konzentration in hochreinem N₂ oder hochreiner Luft enthalten. Es versteht sich, dass bei Messanordnung für andere Wasserinhaltsstoffe entsprechende andere Kalibriergas-Zusammensetzungen einzusetzen sind.

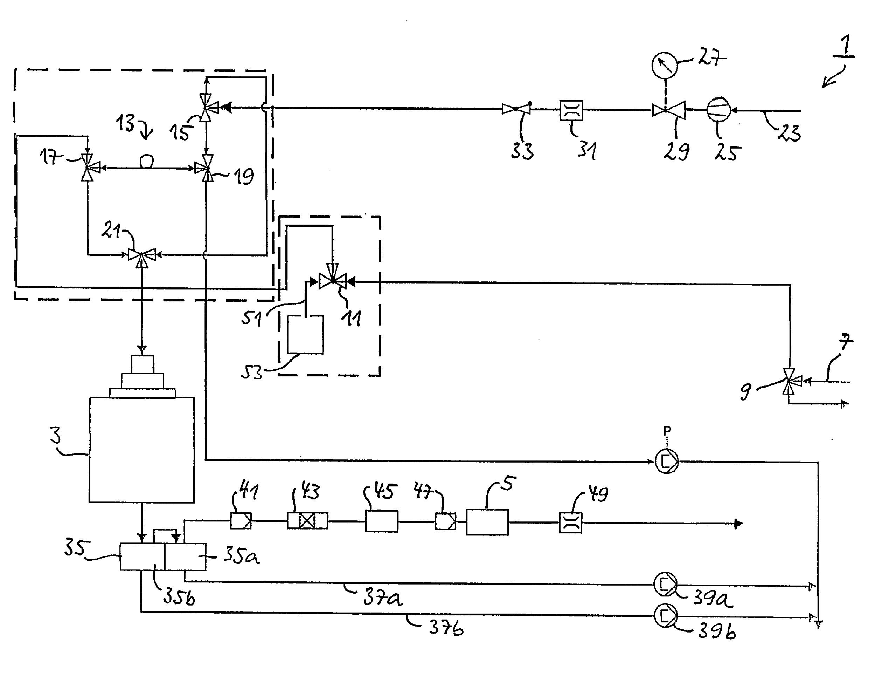

Vorteile und Zweckmäßigkeiten der Erfindung ergeben sich im übrigen aus den abhängigen Ansprüchen sowie der nachfolgenden Beschreibung eines bevorzugten Ausführungsbeispiels anhand der Figur.

Die einzige Figur zeigt eine Prinzipdarstellung einer Ausführungsform der erfindungsgemäßen Anordnung in einem ersten Betriebszustand.

Die Figur zeigt eine TOC-Messanordnung 1 zur Erfassung des Gehaltes einer Prozesswasserprobe an organischem Kohlenstoff, deren Kernstücke ein thermischer Reaktor 3 zum thermischen Aufschluss von Wasserproben und ein Infrarotdetektor 5 zur Erfassung des CO₂-Gehaltes der den Reaktor 3 verlassenden Reaktionsprodukte und somit zur (indirekten) Bestimmung des TOC-Gehaltes in Wasserproben 7 sind. Die Wasserproben 7 gelangen über ein (optionales) Druckreduzierventil 9 und ein Mehrwegeventil 11 zunächst in eine Dosierschleife (Loop) 13 und werden bei einem Messvorgang von dort - in geeigneter Stellung der Mehrwegeventile 15, 17, 19 und 21 - durch einen Transport- bzw. Trägergasstrom 23 in den Reaktor 3 eingetragen und dort schlagartig erhitzt und verbrannt. Der Trägergasstrom 23 wird über eine Förderpumpe 25 und ein mit einer Druckmesseinrichtung 27 ausgestattetes Druckreduzierventil 29 sowie eine Volumenstrom-Messeinrichtung 31 und ein Rückschlagventil 33 zum ersten Mehrwegeventil 15 geführt.

Ausgangsseitig des Reaktors 3 ist zunächst ein Reaktionsgas-Kühler 35 angeordnet, der zwei Stufen 35a, 35b aufweist und aus dem daher zwei Kondenswasserströme 37a, 37b austreten, die jeweils über eine Peristaltik-Pumpe 39a, 39b abgeführt werden. Das insoweit entwässerte Reaktionsgas wird dann über ein Aerosolfilter 41 und eine Säurefalle 43 zu einer Massendurchsatz-Steuerung 45 geführt. Er gelangt von dort schließlich über ein Luftfilter 47 zum bereits erwähnten IR-Detektor 5 und verlässt die Anlage über eine ausgangsseitige Volumenstrom-Erfassungseinrichtung 49.

Anstelle einer als Messprobe dienenden Wasserprobe 7 kann, zur Realisierung eines Kalibrationsvorganges, dem Reaktor ein Kalibriergas 51 im Trägergasstrom 23 zugeführt werden. Zu diesem Zwecke ist an das Mehrwegeventil 11 eine (hier symbolisch dargestellte) Kalibriergasflasche 53 angeschlossen. In einer deren Ausgang mit dem nachgeschalteten Mehrwegeventil 17 verbindenden Stellung des Mehrwegeventils 11 wird dann anstelle einer flüssigen Messprobe 7 eine gasförmige Kalibrierprobe 51 in die Loop 13 eingeleitet. Nachdem diese befüllt ist, kann - analog zum normalen Messvorgang - durch geeignete Stellung der Mehrwegeventil-Gruppe 15, 17, 19 und 21 die Kalibrierprobe in den Reaktor eingetragen werden. Sie wird dort in gleicher Weise wie eine Messprobe umgesetzt, und das Erfassungsergebnis am IR-Detektor 5 wird mit einem auf die gasförmige Kalibrierprobe zugeschnittenen Auswertungsprogramm ausgewertet. Im Ergebnis der Auswertung der Kalibriermessung wird ggf. auch das Auswertungsprogramm für die Messproben-Auswertung zur Kompensation von zwischenzeitlich aufgetretenen Nulllinienverschiebungen o.ä. modifiziert. Damit wird eine gleichbleibende Erfassungsgenauigkeit der TOC-Messanordnung 1 gewährleistet.

Die Ausführung der Erfindung ist nicht auf dieses Beispiel beschränkt, sondern ebenso in einer Vielzahl von Abwandlungen der konkreten Messanordnung wie auch hinsichtlich der zu bestimmenden Wasserinhaltsstoffe und der einzusetzenden (gasförmigen) Kalibrierproben möglich.

## Patentansprüche

1. Verfahren zur Bestimmung eines Wasserinhaltsstoffes, insbesondere des Gehaltes an organischem Kohlenstoff und/oder Stickstoff, bei dem eine wässrige Probe (7) in mindestens einem mit einer Heizeinrichtung versehenen Erhitzungsgefäß (3) verdampft und verbrannt und das Verbrennungsprodukt in einem Transportgasstrom (23) einem Detektor (5) zur Konzentrationsbestimmung einer gasförmigen Verbindung des Wasserinhaltsstoffes zugeführt wird,
wobei eine Kalibrierung mit einer vorbestimmten Menge eines Kalibriergases (51) ausgeführt wird, welches das dem Wasserinhaltsstoff entsprechende Element, insbesondere Kohlenstoff und/oder Stickstoff, in vorbestimmter Konzentration enthält, **dadurch gekennzeichnet, dass** das Kalibriergas (51) anstelle des wässrigen Probe (7) im Transportgasstrom (23) und an der Eingangsseite des Erhitzungsgefäßes (3) zugeführt wird.

2. Verfahren nach Anspruch 1,
**dadurch gekennzeichnet, dass**
zur Bestimmung des Gehaltes von Messproben (7) an organischem Kohlenstoff (TOC) ein Kalibriergas (51) mit einem vorbestimmten CO₂-Gehalt eingesetzt wird.

3. Verfahren nach Anspruch 1 oder 2,
**dadurch gekennzeichnet, dass**
eine vorbestimmte Menge des Kalibriergases (51) durch Befüllung eines Reservoirs, insbesondere Schlauchabschnittes, mit bekanntem Volumen unter Atmosphärendruck oder mit Druckkompensation eingestellt wird, welches nach Befüllung mit dem Kalibriergas (51) vom Transportgasstrom (23) durchströmt wird.

4. Verfahren nach einem der vorangehenden Ansprüche,
**dadurch gekennzeichnet, dass**
im Rahmen einer Kalibrierungsprozedur ein mehrmaliger Eintrag von Kalibriergas in das Verbrennungsgefäß (3), mit jeweiliger Erfassung des Wasserinhaltsstoffes im Detektor (5), erfolgt.

5. Verfahren nach einem der vorangehenden Ansprüche,
**dadurch gekennzeichnet, dass**
die Fläche unter einem Messsignalpeak am Detektor (5) integriert und auf den vorbestimmten Gehalt des Elementes im Kalibriergas (51) normiert wird.

6. Verfahren nach Anspruch 5,
**dadurch gekennzeichnet, dass**
die Normierung unter Einsatz eines vorbestimmten Korrekturfaktors erfolgt.

7. Verfahren nach einem der Ansprüche 4 bis 6,
**dadurch gekennzeichnet, dass**
eine mehrschrittige Kalibrierung mit einer Mehrzahl verschiedener Kalibriergase (51) ausgeführt wird, welche unterschiedliche vorbestimmte Anteile des zu bestimmenden Elementes enthalten.

8. Anordnung zur Durchführung des Verfahrens nach einem der vorangehenden Ansprüche, mit einer Messproben(7)-Zuführeinrichtung, einem Erhitzungsgefäß (3), einer Transportgasquelle, einer ausgangsseitig des Erhitzungsgefäßes (3) angeordneten Detektoreinrichtung (5) und einem das Erhitzungsgefäß (3) eingangsseitig mit der Transportgasquelle verbindenden Strömungsweg, an den die Messproben(7)-Zuführeinrichtung absperrbar angeschlossen ist,
**dadurch gekennzeichnet, dass**
in den Strömungsweg des Transportgasstromes (23) mindestens ein Kalibriergas-Reservoir eingebunden ist, welcher mit einem Kalibriergas (51) befüllt ist, und sich an der Eingangsseite des Erhitzungsgefäßes (3) befindet, so dass das Kalibriergas (51) anstelle der Messprobe (7) zugeführt werden kann.

9. Anordnung nach Anspruch 8,
**dadurch gekennzeichnet, dass**
an den Strömungsweg des Transportgases (23) mindestens eine das Kalibriergas (51), insbesondere ein Kalibriergas (51) mit vorbestimmter CO₂-Konzentration, enthaltende Gasflasche (53) absperrbar angeschlossen ist.

10. Anordnung nach Anspruch 9,
**dadurch gekennzeichnet, dass**
die Gasflasche (53) mit einem Schlauchabschnitt vorbestimmten Volumens verbindbar ist, der einen Abschnitt des Strömungsweges des Transportgases (23) bildet.

11. Anordnung nach Anspruch 9 oder 10,
**dadurch gekennzeichnet, dass**
an den Strömungsweg des Transportgases (23) eine Mehrzahl von das Kalibriergas (51) in unterschiedlichen Konzentrationen enthaltenden Gasflaschen (53) einzeln absperrbar angeschlossen ist.

## Claims

1. A method of determining substances in water, in particular the concentration of organic carbon and/or nitrogen, in which an aqueous sample (7) is evaporated and combusted within at least one heating vessel (3) provided with a heater, and the combustion product is supplied to a detector (5) within a conveying gas flow (23) for determining the concentration of a gaseous compound of the substance in the water,
wherein a calibration is performed with a predetermined amount of a calibrating gas (51) which contains the element corresponding to the substance in the water, in particular carbon and/or nitrogen, in a predetermined concentration,
**characterized in that**
the calibrating gas (51) is supplied within the conveying gas flow (23) and at the inlet side of the heating vessel (3) instead of the aqueous sample (7).

2. The method according to claim 1,
**characterized in that**
a calibrating gas (51) having a predetermined CO₂ concentration is used for determining the content of organic carbon (TOC) in measurement samples (7).

3. The method according to claim 1 or 2,
**characterized in that**
a predetermined amount of the calibrating gas (51) is preset by filling a reservoir, in particular a section of a hose having a known volume, at atmospheric pressure or by pressure compensation, through which flows the conveying gas flow (23) after being filled with the calibrating gas (51).

4. The method according to any one of the preceding claims,
**characterized in that**
the scope of a calibrating procedure encompasses a repeated feeding of calibrating gas into the combustion vessel (3) along with a respective detection of the substance in the water in the detector (5).

5. The method according to any one of the preceding claims,
**characterized in that**
the range below a measurement signal peak at the detector (5) is integrated and standardized to the predetermined concentration of the element in the calibrating gas (51).

6. The method according to claim 5,
**characterized in that**
the standardization is performed using a predetermined correction factor.

7. The method according to any one of claims 4 to 6,
**characterized in that**
a multi-step calibration is performed with a plurality of different calibrating gases (51) containing different predetermined fractions of the element to be determined.

8. An arrangement for performing the method according to any one of the preceding claims comprising a measurement sample (7) supply device, a heating vessel (3), a conveying gas source, a detector device (5) arranged at the outlet side of the heating vessel (3) and a flow path connecting the heating vessel (3) at the inlet side with the conveying gas source to which the measurement sample (7) supply device is connected so as to be able to be blocked,
**characterized in that**
at least one calibrating gas reservoir is integrated in the flow path of the conveying gas flow (23), same being filled with a calibrating gas (51) and situated at the inlet side of the heating vessel (3) so that the calibrating gas (51) can be supplied instead of the measurement sample (7).

9. The arrangement according to claim 8,
**characterized in that**
at least one gas bottle (53) containing the calibrating gas (51), in particular a calibrating gas (51) having a predetermined CO₂ concentration, is connected to the flow path of the conveying gas (23) so as to be able to be blocked.

10. The arrangement according to claim 9,
**characterized in that**
the gas bottle (53) is connectable to a hose section of a predetermined volume, which forms a section of the flow path of the conveying gas (23).

11. The arrangement according to claim 9 or 10,
**characterized in that**
a plurality of gas bottles (53) containing the calibrating gas (51) in different concentrations can be connected to the flow path of the conveying gas (23) so as to be able to be individually blocked.

## Revendications

1. Procédé destiné à déterminer un constituant de l'eau, en particulier la teneur en carbone organique et/ou en azote, où un échantillon aqueux (7) est évaporé et brûlé dans au moins un récipient de chauffe (3) pourvu d'un dispositif de chauffage et où le produit de combustion est amené à un détecteur (5) dans un flux de gaz de transport (23) afin de déterminer la concentration d'une liaison gazeuse du constituant de l'eau,
un calibrage étant effectué avec une quantité prédéterminée d'un gaz de calibrage (51), lequel contient, en concentration prédéterminée, l'élément correspondant au constituant de l'eau, en particulier du carbone et/ou de l'azote,
**caractérisé en ce que** le gaz de calibrage (51) est amené à la place de l'échantillon aqueux (7) dans le flux de gaz de transport (23) et à l'entrée du récipient de chauffe (3).

2. Procédé selon la revendication 1,
**caractérisé en ce que**
un gaz de calibrage (51) ayant une teneur prédéterminée en CO₂ est utilisé pour déterminer la teneur d'échantillons de mesure (7) en carbone organique (TOC).

3. Procédé selon la revendication 1 ou 2,
**caractérisé en ce que**
une quantité prédéterminée du gaz de calibrage (51) est réglée par remplissage d'un réservoir, en particulier d'une section de tuyau, moyennant un volume connu à la pression atmosphérique ou avec compensation de pression, lequel est traversé par le flux de gaz de transport (23) après remplissage moyennant le gaz de calibrage (51).

4. Procédé selon l'une des revendications précédentes,
**caractérisé en ce que**
dans le cadre d'une procédure de calibrage, un chargement répété de gaz de calibrage dans le récipient de combustion (3) est effectué, le constituant de l'eau étant à chaque fois mesuré dans le détecteur (5).

5. Procédé selon l'une des revendications précédentes,
**caractérisé en ce que**
la surface sous un pic de signal de mesure au niveau du détecteur (5) est intégrée et normalisée à la teneur prédéterminée de l'élément dans le gaz de calibrage (51).

6. Procédé selon la revendication 5,
**caractérisé en ce que**
la normalisation est effectuée moyennant un facteur de correction prédéterminé.

7. Procédé selon l'une des revendications 4 à 6,
**caractérisé en ce que**
un calibrage en plusieurs étapes est effectué avec une pluralité de gaz de calibrage (51) divers, lesquels contiennent différentes fractions prédéterminées de l'élément à déterminer.

8. Arrangement permettant d'exécuter le procédé selon l'une des revendications précédentes, comportant un dispositif d'amenée d'échantillons de mesure (7), un récipient de chauffe (3), une source de gaz de transport, un dispositif de détecteur (5) disposé en sortie du récipient de chauffe (3) et une voie d'écoulement reliant le récipient de chauffe (3) en entrée à la source de gaz de transport et à laquelle le dispositif d'amenée d'échantillons de mesure (7) est raccordé de manière à pouvoir être isolé,
**caractérisé en ce que**
au moins un réservoir de gaz de calibrage, lequel est rempli d'un gaz de calibrage (51) et se situe à l'entrée du récipient de chauffe (3), est intégré dans la voie d'écoulement du flux de gaz de transport (23) de sorte que le gaz de calibrage (51) puisse être amené à la place de l'échantillon de mesure (7).

9. Arrangement selon la revendication 8,
**caractérisé en ce que**
au moins une bouteille de gaz (53) contenant le gaz de calibrage (51), en particulier un gaz de calibrage (51) ayant une concentration prédéterminée en CO₂, est raccordé à la voie d'écoulement du gaz de transport (23) de manière à pouvoir être isolée.

10. Arrangement selon la revendication 9,
**caractérisé en ce que**
la bouteille de gaz (53) peut être reliée à une section de tuyau de volume prédéterminé qui forme une section de la voie d'écoulement du gaz de transport (23).

11. Arrangement selon la revendication 9 ou 10,
**caractérisé en ce que**
une pluralité de bouteilles de gaz (53) contenant le gaz de calibrage (51) en différentes concentrations est raccordée à la voie d'écoulement du gaz de transport (23) de manière à pouvoir être isolée individuellement.
